# EUROPEAN PATENT APPLICATION

(11) **EP 1 911 465 A1**
(43) Date of publication of application: **16.04.2008**
(21) Application number: 06021438.4
(22) Date of filing: 12.10.2006
(51) Int. Cl.: A61L 9/14, B05B 7/06

(54) **Method for distributing a disinfectant**

(71) Applicant: Linde Aktiengesellschaft, 65189 Wiesbaden (DE)
(72) Inventor: Falldin, Anders, 16836 Bromma (SE); Marcusson, Anders, 18181 34 Lidingö (SE)

(57) **Abstract**

The invention relates to a method to distribute a disinfectant, wherein an inert gas stream is formed by expansion of a liquid gas and said disinfectant is added to said inert gas stream after said expansion of said liquid gas.

## Description

The invention relates to a method to distribute a disinfectant.

It is an object of the present invention to provide a method to be able to disinfect large areas within a short period of time. The amount of disinfectant should be reduced as far as possible.

This object is achieved by a method to distribute a disinfectant, wherein an inert gas stream is formed by expansion of a liquid gas and wherein said disinfectant is added to said inert gas stream after said expansion of said liquid gas.

The basic idea is to produce a high velocity gas jet, adding the disinfection liquid to the gas jet and spreading it as an aerosol. Therefore, according to the invention a gas is expanded in a nozzle to produce a high velocity gas stream followed by the introduction of the disinfectant into that gas stream. The high speed of the gas stream causes the liquid disinfectant to be divided into small droplets forming a high velocity mist. Due to the small size of the disinfectant droplets, they will follow the highly turbulent inert gas stream around distant corners of the area to be disinfected.

Preferably a gas in the liquid phase is supplied to the nozzle in order to maximize the gas flow. A benefit of the inventive mixing in front of the nozzle is that the gas flow and the disinfectant flow can be varied and turned off independently of each other. Thereby, the inert gas-disinfectant-ratio can be changed easily.

Since the disinfectant is carried by the gas stream it can permeate and expand into all or most of the space or volume to be disinfected and thus provide a total flooding capability.

It is advantageous to form the gas stream by the expansion of liquid nitrogen or liquid carbon dioxide. High pressure gas, in particular high pressure gaseous nitrogen or high pressure gaseous carbon dioxide, can also be used. However, when the gas is not stored at the desired high pressure a compressor would be necessary and the whole system would be less mobile than a disinfection system using a liquid or liquefied gas. In principle high pressure air may also be utilized as propelling medium for the disinfectant.

In particular the use of liquid carbon dioxide is preferred. Liquid carbon dioxide has the advantage that it can be easily stored in high pressure cylinders or tanks. And compared to other gases as liquid nitrogen or liquid argon it is quite easy to keep the liquid carbon dioxide more or less sub cooled in the whole liquid gas supply line between the liquid gas supply and the expansion nozzle, which is necessary in order to get a high flow of gas into the expansion nozzle.

It is advantageous to form a high velocity gas stream, in particular to form a gas stream having supersonic speed. This is preferably achieved by introducing a liquid gas into a nozzle designed to increase the gas velocity when the gas is expanding in the outlet of the nozzle. In particular, the use of a conical nozzle or a Laval nozzle is preferred.

Often it is necessary not to throw the disinfectant over large distances but to spread out the disinfectant, for example to disinfect a volume like a room or a car. This could be achieved by a spray distributor placed in front of the nozzle outlet. The distributor may be permanently fixed to the nozzle or better be a movable part of the nozzle which could be moved in front of the nozzle when needed. The distributor may be arranged to deflect the gas stream only or to deflect the combined gas and disinfectant stream.

The nozzle can be further improved in several ways: The nozzle can be provided with valves, for example manual valves, for the gas as well as for the disinfectant stream or with handles to carry the nozzle and to support the personnel during use. Larger nozzles could be used together with some dedicated mechanical equipment as for example a steering-arm operated from a control box.

For the supply of the liquid inert gas a gas cylinder carried as a backpack is preferably used. Such a supply system is highly mobile and can be moved around easily.

When using a liquid gas supply it is preferred to control the pressure of the liquid gas during its feed to the nozzle in order to maximize the flow rate through the liquid gas supply line. For example a non-condensed gas is used to keep the pressure in the liquid gas supply system stable and also to sub cool more or less all the way from the liquid gas supply to the nozzle. When using liquid carbon dioxide as the propelling medium it is advantageous to pressurize the liquid carbon dioxide storage vessel with gaseous nitrogen.

Since nitrogen is to some extent soluble in carbon dioxide which might lead to a two-phase-flow in the liquid gas supply line, it is preferred to connect at least two carbon dioxide storage vessels in series and to enter the gaseous nitrogen at one end of the combined storage vessels and to take out the liquid carbon dioxide at the opposite end. Thus the carbon dioxide will be pressurized and sub cooled during most of the emptying process of the storage vessels and less nitrogen is dissolved in the carbon dioxide.

In a preferred embodiment of the invention the liquid gas is stored in high pressure storage vessels. The pressure in these vessels can be as high as 190 bars. Normally such high pressure vessels are not provided with any insulation. A single storage vessel will improve the total weight of the gas supply system, but one drawback is the above described risk that nitrogen used for sub cooling will be dissolved in the liquid gas. In practice it depends on the situation whether it is better to use a single storage vessel or a bundle of for example gas cylinders.

Instead of using nitrogen to pressurize the liquid carbon dioxide it is also possible to sub cool the liquid carbon dioxide by helium. If using a single storage vessel it is also possible to use a pump to feed the liquid gas from the storage vessel to the nozzle. The pump could be used for un-insulated tanks but also for insulated tanks in which the liquid carbon dioxide is stored at a lower temperature. The pump should give such a high pressure that during the way through the liquid gas supply line as little as possible of the gas is vaporized. Otherwise a two-phase-flow will occur and the mass flow in the liquid gas supply line will be decreased. Of course such a large storage vessel could also be used with two or more liquid gas supply lines.

The invention has several advantages compared to the prior art methods, especially when large quantities of disinfectant are to be distributed. The inventive method and system can be used at short and long distances, even if it is not possible to come close to the area which shall be disinfected. The inventive method can for example be used to disinfect cars or for nuclear decontamination.

The inventive method to spray a mixture of small droplets of a liquid disinfectant and a high velocity gas stream is very efficient. The time for disinfecting a particular area is essentially reduced. By distributing the disinfectant as an aerosol the amount of disinfectant needed to disinfect an area or an object is also reduced.

Another benefit of the invention is the possibility to switch between different disinfection agents. Further the amount of disinfectant mixed into the gas stream can easily be changed.

The invention as well as further details and preferred embodiments of the invention are disclosed in the following description and illustrated in the accompanying drawings, in which
figure 1 schematically shows a disinfecting system according to the invention, and
figure 2 an inventive nozzle.

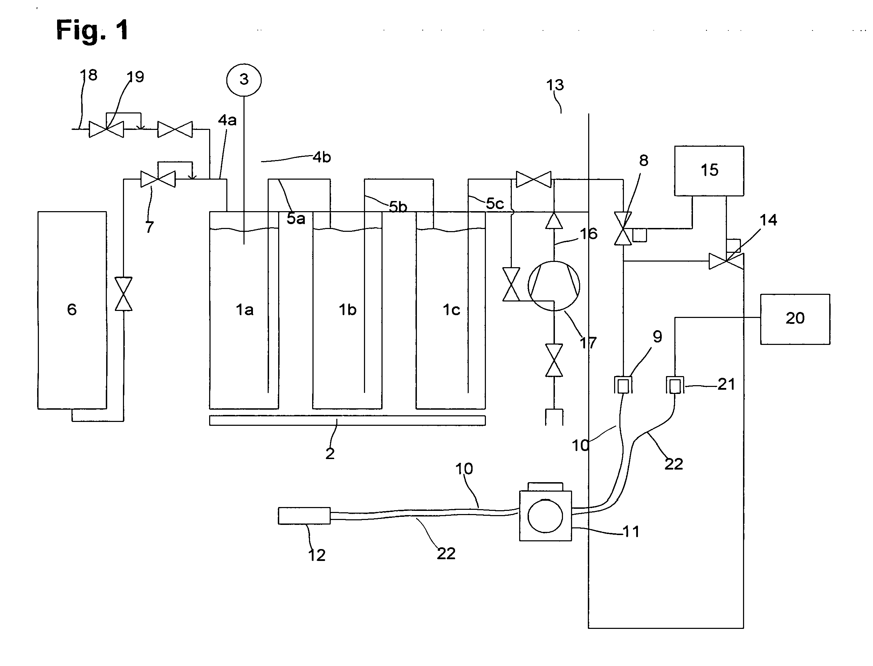

The invention is preferably carried out using carbon dioxide as gas. The carbon dioxide and the disinfectant supply for such a system are shown in figure 1.

Liquid carbon dioxide is stored in three high pressure uninsulated storage vessels 1a, 1b, 1 c. Typically about 1500 kg of liquid carbon dioxide is stored in storage vessels 1 a, 1 b, 1 c. The storage vessels 1 a, 1 b, 1 c are mounted on a scale 2 which is used to determine the amount of carbon dioxide stored in the storage vessels 1a, 1b, 1c. In addition the storage vessels 1 a, 1b, 1 c are provided with liquid level indicators 3. When filling or re-filling the storage vessels 1a, 1b, 1c with liquid carbon dioxide the liquid indicators 3 and/or the scale 2 are used to stop the filling procedure when a predetermined liquid level respectively predetermined weight has been reached.

Storage vessel 1 a is provided with tubes 4a, 4b connected to the gas phase of the stored carbon dioxide. Tubes 5a, 5b, 5c are extending into the liquid phase of each storage vessel 1 a, 1 b, 1 c. The liquid phases of storage vessels 1 a, 1 b, 1 c are connected in series, i. e. liquid tube 5a connects the liquid phases in storage vessels 1a and 1b, and liquid tube 5b the liquid phases in storage vessels 1b and 1c.

Tube 4a connected to the gas phase of storage vessel 1a is further connected to a gas cylinder 6 filled with gaseous nitrogen at a pressure of about 200 bars. Tube 4a is provided with a pressure regulated valve 7 having a set pressure of about 5 to 10 bars above the boiling point of the liquid carbon dioxide in the storage vessels 1a, 1b, 1c.

Instead of the serial connection of storage vessels 1a, 1b, 1c it is also possible to connect them in parallel or to use one single storage vessel. It is also an advantage to use helium instead of nitrogen, but due to the higher price of helium nitrogen might still be preferred. Further instead of nitrogen cylinder 6 a liquid carbon dioxide pump located in liquid tube 5c may be used to increase the pressure of the liquid carbon dioxide and to pump the liquid carbon dioxide from the storage vessel 1 a, 1 b, 1 c to the nozzle 12. It will be apparent to one skilled in the art that then the piping has to be changed somewhat.

Liquid tube 5c extending into the liquid phase of storage vessel 1c is at its other end provided with an outlet valve 8 and a connection point 9 which is used for connecting a liquid carbon dioxide hose 10. Liquid carbon dioxide hose 10 is arranged in a hose cassette 11 and at its end provided with an ejector 12.

Gas tube 4a leads to a gas line 13 which is connected to the liquid tube 5c downstream outlet valve 8. The gas flow through gas line 13 can be regulated by means of gas valve 14. Gas valve 14 as well as outlet valve 8 can be controlled by an operating system 15. Valve 14 is used to pressurize the carbon dioxide line and the liquid carbon dioxide hose 10 in order to avoid dry-ice formation in the line.

To refill the liquid carbon dioxide storage vessels 1 a, 1b, 1 c a fill line 16 comprising a fill pump 17 is connected to liquid tube 5c. To avoid over-pressure during the filling of the storage vessels 1 a, 1b, 1 c a venting line 18, which is provided with a filling regulator 19, branches from the gas tube 4a.

However, it will be apparent to one skilled in the art that the liquid gas supply system and liquid supply line could be arranged in other versions that depart from these specific details.

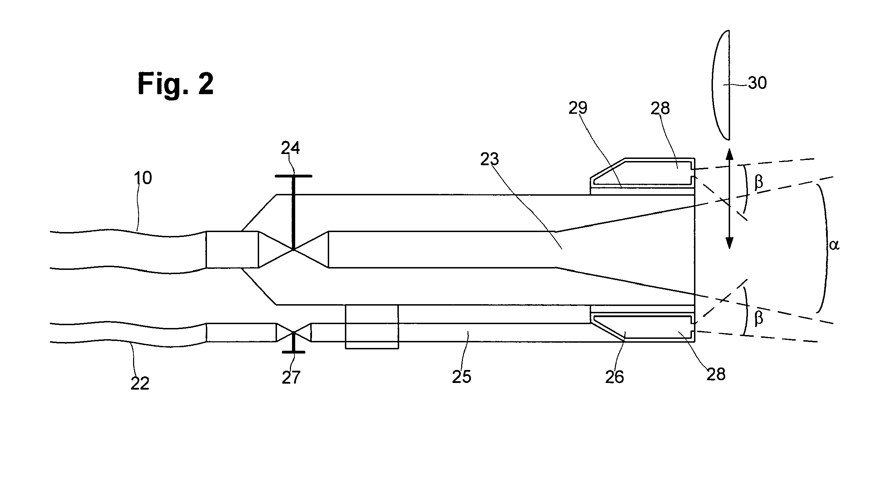

A liquid disinfectant is supplied for example from a tank 20, and if necessary a pump, not shown in figure 1, is used to increase the feed pressure. At connection point 21 a disinfectant hose 22 is connected to the disinfectant supply system. Disinfectant hose 22 and carbon dioxide hose 10 are bound together. Disinfectant hose 22 also leads to the ejector 12 which in the following will be described with reference to figure 2.

In figure 2 the carbon dioxide and disinfectant ejector 12 is shown in greater detail. Liquid carbon dioxide hose 10 is connected to a nozzle 23. The liquid carbon dioxide flow to nozzle 23 can be manually regulated by valve 24. Nozzle 23, for example a Laval nozzle, is designed to achieve a gas stream of high velocity, especially to increase the gas velocity above sonic speed.

Disinfectant hose 22 is connected via tube 25 to a disinfectant distributor 26. The disinfectant flow can also be regulated by using disinfectant valve 27. The disinfectant is transferred through tube 25 to the disinfectant distributor 26 which surrounds nozzle 23 and which has several outlet openings 28. Insulation 29 is provided between the nozzle 23 and the disinfectant distributor 26 to avoid freezing of the disinfectant.

A spray distributor 30 is movably arranged in front of the outlet of the nozzle 23. If necessary the spray distributor 30 can be moved directly in front of nozzle 23 and thus deflect the carbon dioxide gas jet in order to create a broad gas stream.

In the following a preferred mode of operation of the invention is described. The system shown in figures 1 and 2 is mounted on a truck. Vessels 1a, 1b, 1c are filled with liquid carbon dioxide. Therefore, a source of liquid carbon dioxide is connected to the fill line 16. Fill pump 17 is started and liquid carbon dioxide is pumped through liquid tubes 5c, 5b, 5a into storage vessels 1 a, 1 b, 1 c. The filling procedure is stopped when scale 2 or liquid level indicator 3 show a predetermined weight or predetermined liquid level indicating that storage vessels 1a, 1b, 1c are completely filled. In case the pressure within storage vessels 1 a, 1b, 1 c increases above the set value of filling regulator 19, filling regulator 19 opens and gaseous carbon dioxide is blown via venting line 18 to the atmosphere.

For distributing the disinfectant carbon dioxide hose 10 and disinfectant hose 22 are connected to the respective connection points 9 and 21 on the truck and hose cassette 11 is wheeled away in the direction to the area which shall be disinfected. When being close enough to the area to be disinfected, ejector 12 is connected to hoses 10, 22.

Operating system 15 opens valve 14 to allow gaseous carbon dioxide to flow into carbon dioxide hose 10 in order to pressurise the hose 10. Then valve 14 is closed and the liquid carbon dioxide valve 8 is opened. Nitrogen gas is fed from nitrogen cylinder 6 into the storage vessels 1a, 1b, 1c at a pressure of about 5 bars above the boiling point of liquid carbon dioxide. Pressure regulator 7 ensures that during emptying the storage vessels 1 a, 1 b, 1 c the gas pressure is kept stable.

The gaseous nitrogen pushes liquid carbon dioxide out of storage vessels 1 a, 1b, 1 c via liquid tube 5c and hose 10 to the carbon dioxide ejector 12. Due to the serial connection of storage vessels 1 a, 1 b, 1 c and due to the fact that gaseous nitrogen enters the storage vessels at one end and the liquid carbon dioxide is withdrawn at the opposite end, the amount of nitrogen fed into liquid tube 5c and hose 10 is minimized.

Disinfectant is supplied from tank 20 via disinfectant hose 22 to the ejector 12, either by use of a high pressure pump not shown in figure 1 or any other suitable means.

At the ejector 12 valve 24 is opened and liquid carbon dioxide flows into nozzle 23. The liquid carbon dioxide expands in nozzle 23, forms a mixture of gaseous, liquid and solid carbon dioxide and leaves the nozzle 23 with very high speed.

Disinfectant valve 27 is opened and disinfectant flows into disinfectant distributor 26 which is coaxially arranged with nozzle 23. The disinfectant is pushed out through outlet openings 28, thus creating disinfectant jets surrounding the central high speed carbon dioxide jet. The disinfectant jets and the carbon dioxide leave ejector 12 as essentially parallel gas streams. However, for certain applications it might be advantageous to adjust the ejection angle of the disinfectant and/or the carbon dioxide jet as well as their respective angles α, β of jet spread.

The disinfectant is sprayed into the carbon dioxide jet and by the large speed of the carbon dioxide the disinfectant is divided into fine droplets forming a high speed carbon dioxide-disinfectant-mist which has a long throwing length.

## Claims

1. Method to distribute a disinfectant, **characterized in that** an inert gas stream is formed by expansion of a liquid gas and that said disinfectant is added to said inert gas stream after said expansion of said liquid gas.

2. Method according to claim 1, **characterized in that** said disinfectant is substantially liquid at ambient temperature and pressure.

3. Method according to any of claims 1 or 2, **characterized in that** said inert gas stream is formed by expansion of liquid carbon dioxide.

4. Method according to any of claims 1 to 3, **characterized in that** an inert gas stream is formed having supersonic speed.

5. Method according to any of claims 1 to 4, **characterized in that** prior to its expansion said liquid gas is pressurized to a pressure above the boiling point of said liquid gas.

6. Method according to any of claims 1 to 5, **characterized in that** said liquid gas is pressurized with gaseous nitrogen or gaseous helium.
